# EUROPEAN PATENT APPLICATION

(11) **EP 4 516 360 A1**
(43) Date of publication of application: **05.03.2025**
(21) Application number: 23796151.1
(22) Date of filing: 14.04.2023
(51) Int. Cl.: A61Q 1/00, A61Q 19/00, A61K 8/49

(54) **COSMETIC**

(30) Priority: 25.04.2022 JP 2022071758
(71) Applicant: Shiseido Company, Ltd., Chuo-ku Tokyo 104-0061 (JP)
(72) Inventor: FUNAYAMA Mayu, Tokyo 104-0061 (JP)
(74) Representative: Ter Meer Steinmeister & Partner
(86) International application number: PCT/JP2023/015169
(87) International publication number: WO 2023/210408

(57) **Abstract**

The present invention provides a cosmetic capable of achieving both an effect brought about by nicotinic acid or a derivative thereof and a feeling of use. The cosmetic according to the present invention is a cosmetic containing a powder of nicotinic acid or a derivative thereof having an average particle diameter of 40 µm or less, and is a cosmetic containing a powder of nicotinic acid or a derivative whose content is 3% by mass or more based on the total mass of the cosmetic.

## Description

### TECHNICAL FIELD

The present invention relates to a cosmetic.

### BACKGROUND ART

Niacinamide is known to be an effective component for improving stains and wrinkles. In addition, Niacinamide is also known to be effective as a blood circulation promoter and a hair activator. Therefore, niacinamide is widely used in various cosmetics. Niacinamide generally has a powder shape, but has high solubility in water or ethanol, and therefore is often dissolved in a liquid base to be used in a cosmetic.

Meanwhile, some cosmetics are in a formulation form that hardly contains a solvent or the like, such as a powder foundation or a concealer containing powder. It is difficult to blend niacinamide in a dissolved state in such a cosmetic. Therefore, in order to blend niacinamide in such a cosmetic, niacinamide in a solid state (powder state) is blended. However, according to studies of the present inventors, it has been found that a cosmetic containing niacinamide in a solid state, which has been generally used heretofore, has a poor feeling of use when brought into contact with the skin. The same applies to cosmetics in which powders other than niacinamide are blended, and it has been found that even when niacinamide in a solid state is blended in a cosmetic in which a silica powder or a resin powder is blended, a feeling of use tends to deteriorate.

Even in prior art, a cosmetic containing niacinamide in a solid state is hardly known. As far as the present inventors know, there are only several known cosmetics in which niacinamide is blended in a very small amount of about 0.1% by weight with respect to the total mass of a cosmetic (for example, Patent Literature 1).

### CITATION LIST

### PATENT LITERATURE

Patent Literature 1: JP 2010-503724 A

### SUMMARY OF THE INVENTION

The present invention provides the following inventions.
[1] A cosmetic containing a powder of nicotinic acid or a derivative thereof having an average particle diameter of 40 µm or less.
[2] The cosmetic according to [1], in which the content of the powder is 3% by mass or more based on the total mass of the cosmetic.
[3] The cosmetic according to [1] or [2], in which the nicotinic acid or a derivative thereof is selected from nicotinic acid, niacinamide, benzyl nicotinate, tocopherol nicotinate, and a nicotinic acid β-butoxy ester.
[4] The cosmetic according to [1] or [2], further containing an inorganic powder different from the powder.
[5] The cosmetic according to [1] or [2], in which the content of water is 1% by mass or less based on the total mass of the cosmetic.
[6] The cosmetic according to [1] or [2], in which the cosmetic is in a form of a powder solid cosmetic or a powder cosmetic.
[7] A cosmetic containing a powder of nicotinic acid or a derivative thereof, in which the content of the powder is 3% by mass or more based on the total mass of the cosmetic.
[8] A method for manufacturing a cosmetic containing a powder of nicotinic acid or a derivative thereof having an average particle diameter of 40 µm or less, the method including
   pulverizing particles of nicotinic acid or a derivative thereof having an average particle diameter of more than 40 µm to form a powder having an average particle diameter of 40 µm or less.
[9] The method for manufacturing a cosmetic according to [8], in which inorganic particles different from the particles are mixed with the particles, and then the obtained mixture is pulverized.

By containing a powder of nicotinic acid or a derivative thereof, the cosmetic according to the present invention can achieve an excellent feeling of use while having an effect brought about by nicotinic acid or a derivative thereof in a cosmetic containing almost no liquid, such as powder.

### DETAILED DESCRIPTION OF THE INVENTION

### [First embodiment]

A cosmetic according to a first embodiment of the present invention contains a powder of nicotinic acid or a derivative thereof having an average particle diameter of 40 µm or less.

Examples of the derivative of nicotinic acid include niacinamide, benzyl nicotinate, tocopherol nicotinate, and a nicotinic acid β-butoxy ester, and niacinamide is preferable among these derivatives. Here, niacinamide is also referred to as nicotinamide or niacinamide, and is an amide compound of nicotinic acid (vitamin B3/niacin). Niacinamide is a known substance that is a water-soluble vitamin belonging to a vitamin B group, may be extracted from a natural product (rice bran or the like) or can be synthesized by a known method. For example, those listed in the Japanese Pharmacopoeia, Eighteenth Edition can be used.

In general, nicotinic acid or a derivative thereof is blended in a cosmetic in a state of being dissolved in a solvent such as water. On the other hand, in the cosmetic according to the present invention, nicotinic acid or a derivative thereof is blended in a powder state. Here, nicotinic acid or a derivative thereof, for example, niacinamide used industrially is generally commercially available in a particle form. This is considered to be because when niacinamide has an excessively small average particle diameter, scattering or the like is likely to occur, and handleability is deteriorated. Specifically, niacinamide used industrially generally has an average particle diameter of more than 40 µm.

On the other hand, the average particle diameter of nicotinic acid or a derivative thereof used in the present invention is 40 µm or less, preferably 30 µm or less, and more preferably 15 µm or less. By using nicotinic acid or a derivative thereof having such a small average particle diameter, the cosmetic according to the present invention can achieve both an effect of nicotinic acid or a derivative thereof as a drug and an excellent feeling of use. A material having such a small average particle diameter can be obtained by pulverizing a commercially available material (details will be described later). Note that, in the present invention, a material before pulverization may be referred to as a particle, and a material having a small average particle diameter after pulverization may be referred to as a powder.

In the present invention, the average particle diameter of a powder can be measured by a laser diffraction/scattering method. The "average particle diameter" in the present invention is a particle diameter at which a cumulative frequency is 50% from a number-based particle size distribution curve measured by a laser diffraction/scattering method, particularly a microtrack laser diffraction method. In the microtrack laser diffraction method, scattered light when powder particles are irradiated with laser light is used.

More specifically, the average particle diameter is measured by a wet method in which a solvent that does not dissolve nicotinic acid or a derivative thereof, for example, niacinamide, for example, a solvent such as an isoparaffinic hydrocarbon solvent (for example, ISOPAR (trade name, manufactured by Exxon Mobil Corporation) or D-5 is circulated, and a powder sample is put into the solvent and irradiated with laser light. The measurement can be performed using, for example, Microtrac MT-3000 manufactured by Nikkiso Co., Ltd. According to this method, in general, measurement can be performed for a powder having an average particle diameter of 0.1 µm to 200 µm.

A blending ratio of nicotinic acid or a derivative thereof contained in the cosmetic of the present invention is not particularly limited, but is preferably 3% by mass or more, and particularly preferably 5% by mass or more with respect to the total mass of the cosmetic. In general, when nicotinic acid or a derivative is used for a cosmetic in a form of particles or powder, a feeling of use tends to be impaired. However, by using nicotinic acid or a derivative thereof having the average particle diameter specified in the present invention, a sufficiently satisfactory feeling of use can be achieved even when the blending ratio is high. Meanwhile, although an upper limit is not particularly limited, it is difficult to obtain an effect proportional to the addition amount even when the blending ratio is excessively increased. Therefore, the blending ratio of nicotinic acid or a derivative thereof is preferably 10% by mass or less, and particularly preferably 8% by mass or less with respect to the total mass of the cosmetic.

The cosmetic according to the invention can contain a component other than nicotinic acid or a derivative thereof. As such a component, first, an inorganic powder different from nicotinic acid or a derivative thereof can be exemplified.

Examples of the inorganic powder include silica (anhydrous silicic acid), talc, boron nitride, sericite, natural mica, calcined mica, synthetic mica, synthetic sericite, alumina, mica, kaolin, bentonite, smectite, calcium carbonate, magnesium carbonate, calcium phosphate, magnesium oxide, tin oxide, iron oxide, yttrium oxide, chromium oxide, titanium oxide, zinc oxide, cerium oxide, aluminum oxide, magnesium oxide, chromium hydroxide, Prussian blue, ultramarine blue, calcium phosphate, aluminum hydroxide, barium sulfate, magnesium sulfate, silicic acid, aluminum magnesium silicate, calcium silicate, barium silicate, magnesium silicate, aluminum silicate, strontium silicate, calcium aluminum borosilicate, silicon carbide, magnesium fluoride, a metal tungstate, magnesium aluminate, magnesium aluminometasilicate, chlorhydroxyaluminum, clay, zeolite, hydroxyapatite, ceramic powder, spinel, mullite, cordierite, aluminum nitride, titanium nitride, silicon nitride, lanthanum, samarium, tantalum, terbium, europium, neodymium, Mn-Zn ferrite, Ni-Zn ferrite, silicone carbide, cobalt titanate, barium titanate, iron titanate, lithium cobalt titanate, cobalt aluminate, antimony-containing tin oxide, tin-containing indium oxide, magnetite, aluminum powder, gold powder, silver powder, platinum powder, copper powder, noble metal colloid, iron powder, zinc powder, cobalt blue, cobalt violet, cobalt green, low order titanium oxide, fine particle titanium oxide, butterfly-shaped barium sulfate, petal-shaped zinc oxide, tetrapod-shaped zinc oxide, fine particle zinc oxide, and as pearl pigments, titanium oxide-coated mica, titanium oxide-coated mica, titanium oxide-coated synthetic mica, titanium oxide-coated silica, titanium oxide-coated synthetic mica, titanium oxide-coated talc, zinc oxide-coated silica, titanium oxide-coated colored mica, red iron oxide-coated titanated mica, red iron oxide/black iron oxide-coated titanated mica, carmine-coated titanated mica, and blue iron-coated titanated mica.

Furthermore, as an inorganic powder functioning as a coloring material, an inorganic red pigment (for example, iron oxide or iron titanate), an inorganic brown pigment (for example, γ-iron oxide), an inorganic yellow pigment (for example, yellow iron oxide or yellow earth), an inorganic black pigment (for example, black iron oxide or low order titanium oxide), an inorganic violet pigment (for example, mango violet or cobalt violet), an inorganic green pigment (for example, chromium oxide, chromium hydroxide, or cobalt titanate), an inorganic blue pigment (for example, ultramarine blue or Prussian blue), a pearl pigment (for example, bismuth oxychloride, fish scale foil, or titanated mica), a metal powder pigment (for example, aluminum powder or copper powder), and the like can also be used. An organic powder can also be used as a coloring material according to a purpose. Examples of such an organic powder include organic pigments of zirconium, barium, and aluminum lake (for example, Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1).

Among these components, silica, talc, mica, sericite, kaolin, titanium oxide, iron oxide, zinc oxide, and the like are preferable, and silica is particularly preferable. This is because when silica is combined with pulverized nicotinic acid or a derivative thereof in a cosmetic containing no solvent, an effect of improving usability tends to be large. This improving effect is strongly exhibited also in a cosmetic in which a resin powder is used as a raw material or removed. These inorganic powders may be subjected to a surface treatment, and are particularly preferably subjected to a hydrophobic treatment. In such a hydrophobic treatment, for example, a hydrophobic treatment agent such as a fatty acid, a higher fatty acid, a higher alcohol, a hydrocarbon, a triglyceride, an ester, a silicone oil, a silicone resin, or a fluorine compound is used.

In addition, these inorganic powders can have various particle shapes, but preferably contain particles having a spherical particle shape. In particular, many spherical porous or non-porous silicas are known, and it is preferable to use these silicas. In addition, particles having a plate shape or a needle shape can also be used, and it is preferable to combine these particles while considering usability and the like.

A blending ratio of the inorganic powder in the cosmetic according to the present invention is preferably 30 to 90% by mass, and preferably 50 to 85% by mass based on the total mass of the cosmetic.

In addition, as described above, the cosmetic according to the present invention can achieve excellent usability even when not containing a resin powder generally used for improving usability, but can contain a resin powder or an organic powder as necessary. For example, the organic pigment described above can be preferably used because it can achieve coloring suitable for a cosmetic, and a cellulose powder, starch, and an amino acid powder (for example, lauroyl lysine) can be preferably used because they can further improve usability. In addition, a resin powder such as a silicone elastomer powder, a silicone powder, a silicone resin-coated silicone elastomer powder, a polyamide resin powder (nylon powder), a polyethylene powder, a polystyrene powder, a styrene-acrylic acid copolymer resin powder, a benzoguanamine resin powder, or a polytetrafluoroethylene powder can also be used, but the content thereof is preferably small, and for example, is typically 5% by mass or less, preferably 3% by mass or less, more preferably 1% by mass or less, and particularly preferably 0% by mass based on the total mass of the cosmetic.

The cosmetic according to the present invention contains nicotinic acid or a derivative thereof and an inorganic powder as main components, but a formulation form thereof is preferably a powder solid cosmetic or a powder cosmetic. This is because a feeling of use improving effect according to the present invention is remarkably exhibited when the cosmetic is in such a formulation form. Out of these forms, the powder solid cosmetic is a cosmetic which is made easy to handle by suppressing scattering of a powder, and is obtained by solidifying the powder with a binder or the like. As the binder, an oil is generally used.

The oil that can be used in the cosmetic according to the present invention is not particularly limited as long as it can be generally used in manufacture of a cosmetic. Specific examples thereof include a liquid oil and fat (for example, macadamia nut oil, avocado oil, or camellia oil), a solid oil and fat (for example, cocoa butter, coconut oil, horse fat, or hardened coconut oil), a wax (for example, beeswax, candelilla wax, cotton wax, carnauba wax, or bayberry wax), a hydrocarbon oil (for example, liquid paraffin, ozokerite, or squalane), a higher fatty acid (for example, lauric acid, myristic acid, palmitic acid, stearic acid, or behenic acid), a higher alcohol (lauryl alcohol, cetyl alcohol, stearyl alcohol, behenyl alcohol, or butyl alcohol), a synthetic ester oil (for example, isopropyl myristate, cetyl octanoate, octyldodecyl myristate, isopropyl palmitate, or neopentyl glycol dicaprate), and a silicone oil (for example, dimethylpolysiloxane, methylhydrogenpolysiloxane, methylphenylpolysiloxane, or stearoxymethylpolysiloxane).

When an oil is blended, a blending ratio thereof is suitably 1 to 20% by mass, and preferably 5 to 10% by mass with respect to the total mass of the cosmetic.

In addition, the cosmetic according to the present invention can contain other components as long as the effect of the present invention is not impaired. Examples of the other components include an antiseptic (for example, ethylparaben, butylparaben, chlorphenesin, or phenoxyethanol), a dispersant

(various surfactants), an antioxidant (for example, tocopherols, dibutylhydroxytoluene, butylhydroxyanisole, or gallic acid esters), a fragrance, a moisturizer, a water-soluble polymer, a thickener, a coating agent, an ultraviolet absorber, a sequestering agent, a lower alcohol, a polyhydric alcohol, a sugar, an amino acid, an organic amine, a polymer emulsion, a pH adjuster, a skin nutrient, a vitamin, and water.

Note that, in the cosmetic according to the present invention, the content of water is particularly preferably zero in order to maximize the effect obtained by nicotinic acid or a derivative thereof blended as a powder. Therefore, when water is blended as necessary, a blending ratio thereof is preferably 1% by mass or less, and more preferably 0.5% by mass or less based on the total mass of the cosmetic.

The cosmetic according to the present invention is preferably in a form of a powder solid cosmetic or a powder cosmetic, but specifically, more preferably in a form of a foundation, an eye shadow, a cheek color, a body powder, a perfume powder, a baby powder, a pressed powder, a deodorant powder, a face powder, or the like.

### [Second embodiment]

A cosmetic according to a second embodiment of the present invention is a cosmetic containing a powder of nicotinic acid or a derivative thereof, in which a content of the powder is 3% by mass or more based on the total mass of the cosmetic.

As described above, when nicotinic acid or a derivative thereof is blended in a cosmetic, nicotinic acid or a derivative thereof is often blended in a state of being dissolved in a solvent, and nicotinic acid or a derivative thereof in a solid state is hardly blended in a powder cosmetic. This is presumed to be because it is considered to be efficient to bring nicotinic acid or a derivative thereof into contact with the skin in a state of being dissolved in a solvent such as water in order to obtain drug efficacy of nicotinic acid or a derivative thereof. However, when a cosmetic using nicotinic acid or a derivative thereof in a powder state is brought into contact with the skin, there is also an influence of moisture or the like supplied from the skin, and therefore, even a powder cosmetic containing no water can be expected to obtain drug efficacy thereof.

That is, in the cosmetic according to the present invention, the content of the powder of nicotinic acid or a derivative thereof is 3% by mass or more, and preferably 5% by mass or more based on the total mass of the cosmetic.

Such a cosmetic containing nicotinic acid or a derivative thereof at a high blending ratio can be manufactured by, for example, blending a powder of nicotinic acid or a derivative thereof having a small average particle diameter, pulverizing and then blending particles of nicotinic acid or a derivative thereof as a raw material, or pulverizing a raw material mixture (details will be described later).

The cosmetic according to the second embodiment can combine components similar to those of the cosmetic according to the first embodiment except that the cosmetic according to the second embodiment contains nicotinic acid or a derivative thereof at a high blending ratio.

### [Third embodiment]

A method for manufacturing a cosmetic according to a third embodiment of the present invention is a method for manufacturing a cosmetic containing a powder of nicotinic acid or a derivative thereof having an average particle diameter of 40 µm or less. This manufacturing method includes pulverizing particles of nicotinic acid or a derivative thereof having an average particle diameter of more than 40 µm to form a powder having an average particle diameter of 40 µm or less.

When the particles are pulverized, only nicotinic acid or a derivative thereof may be pulverized, or a mixture containing other components to be blended in the cosmetic, for example, inorganic particles may be pulverized. For the pulverization, a stirring mixer or a pulverizing machine, such as a Henschel mixer, an atomizer, a Nauta mixer, a ribbon blender, a kneader, or a pulverizer can be used.

When only particles of nicotinic acid or a derivative thereof, for example, only niacinamide particles are pulverized to form a powder, it is preferable to use an atomizer or the like capable of forming a powder having a relatively small average particle diameter. In addition, it is also preferable to use an atomizer after pulverization with a Henschel mixer or the like capable of forming a powder having a relatively large average particle diameter.

When pulverization is performed after nicotinic acid or a derivative thereof is mixed with components, it is common to first use a Henschel mixer for pulverization. Since an inorganic powder and the like are blended as other components, nicotinic acid or a derivative thereof tends to have an average particle diameter smaller by contact with the inorganic powder than that of a case in which nicotinic acid or a derivative thereof is pulverized alone. However, when the average particle diameter of nicotinic acid or a derivative thereof does not become, for example, 40 µm or less, it is preferable to further pulverize nicotinic acid or a derivative thereof with an atomizer or the like. Note that the average particle diameter of all the materials contained in the pulverized cosmetic does not need to be 40 µm or less. Even when the average particle diameter of components other than nicotinic acid or a derivative thereof exceeds 40 µm, an influence on a feeling of use is small.

When an oil is blended as a component to form a powder solidified cosmetic, it is also possible to blend the materials stepwise in addition to mixing and pulverizing all the materials first. Particularly preferably, the pulverization step is divided into two or more steps, and an oil is blended after one or more pulverization steps are performed. Specifically, a mixture obtained by mixing and pulverizing nicotinic acid or a derivative thereof, an inorganic powder, and if necessary, a component other than an oil among components of other materials with a Henschel mixer, and then mixing the oil can be pulverized with an atomizer. Through such a step, a uniform powder cosmetic containing no coarse particles can be obtained. The powder cosmetic after the pulverization can be compressed by a compressor or the like to form a powder solid cosmetic.

### EXAMPLES

### [Examples 1 to 5]

A powder solid cosmetic (pressed powder cosmetic) was prepared with a formulation presented in Table 1, and performance thereof was evaluated. Evaluation criteria are as follows.

### Roughness

A tester evaluated a feeling of roughness when a cosmetic was applied to the skin according to the following criteria.
S: It was felt that there was no roughness.
A: It was felt that there was almost no roughness.
B: It was felt that there was slight roughness.
C: It was felt that there was roughness.

### Smoothness

The tester evaluated a feeling of smooth when a cosmetic was applied to the skin according to the following criteria.
S: It was felt to be very smooth.
A: It was felt to be sufficiently smooth.
B: It was felt to be smooth.
C: It was felt that there was almost no smoothness.

### [Table 1]

**Table 1**

| Component | | Example 1 | Example 2 | Example 3 | Example 4 | Example 5 |
|---|---|---|---|---|---|---|
| Niacinamide | Pulverized product A (average particle diameter: 11 µm) | 5.000 | 2.000 | - | - | - |
| | Commercially available product X (average particle diameter: 55 µm) | - | - | 5.000 | 0.100 | 0.000 |
| Powder | Metal soap hydrophobic treatment talc | Balance | Balance | Balance | Balance | Balance |
| | Hydrophobic treatment mica | 23.000 | 23.000 | 23.000 | 23.000 | 23.000 |
| | Synthetic phlogopite | 15.500 | 15.500 | 15.500 | 15.500 | 15.500 |
| | Lauroyl lysine | 2.000 | 2.000 | 2.000 | 2.000 | 2.000 |
| | Starch | 5.500 | 5.500 | 5.500 | 5.500 | 5.500 |
| | Silica | 1.500 | 1.500 | 1.500 | 1.500 | 1.500 |
| | Red iron oxide | 0.030 | 0.030 | 0.030 | 0.030 | 0.030 |
| | Yellow iron oxide | 0.050 | 0.050 | 0.050 | 0.050 | 0.050 |
| | Chlorphenesin | 0.200 | 0.200 | 0.200 | 0.200 | 0.200 |
| Oil | Dimethicone | 3.000 | 3.000 | 3.000 | 3.000 | 3.000 |
| | Triethylhexanoin | 7.000 | 7.000 | 7.000 | 7.000 | 7.000 |
| | Sorbitan sesquiisostearate | 2.000 | 2.000 | 2.000 | 2.000 | 2.000 |
| Total | | 100.000 | 100.000 | 100.000 | 100.000 | 100.000 |
| Evaluation | Roughness | A | A | C | A | A |
| | Smoothness | A | A | B | A | A |

From the above results, it is found that even when the added amount of niacinamide is increased, use of pulverized niacinamide provides a smooth feeling of use without roughness. In addition, an improving effect by niacinamide tended to be stronger as a blending amount thereof was increased, but it was found that the improving effect was insufficient when the blending amount was about 0.01% by mass with respect to the total mass of a cosmetic, and it was preferable to add a larger amount of niacinamide.

### [Examples 6 to 10]

Another powder solid cosmetic (pressed powder cosmetic) was prepared with a formulation presented in Table 2, and performance thereof was evaluated in a similar manner to Example 1.

### [Table 2]

**Table 2**

| Component | | Example 6 | Example 7 | Example 8 | Example 9 | Example 10 |
|---|---|---|---|---|---|---|
| Niacinamide | Pulverized product A (average particle diameter: 11 µm) | 5.000 | - | - | - | - |
| | Pulverized product B (average particle diameter: 20µm) | - | 5.000 | - | - | - |
| | Pulverized product C (average particle diameter: 40 µm) | - | - | 5.000 | - | - |
| | Commercially available product X (average particle diameter: 55 µm) | - | - | - | 5.000 | - |
| | Commercially available product Y (average particle diameter: 100 µm) | - | - | - | - | 5.000 |
| Powder | Metal soap hydrophobic treatment talc | Balance | Balance | Balance | Balance | Balance |
| | Hydrophobic treatment mica | 15.500 | 15.500 | 15.500 | 15.500 | 15.500 |
| | Synthetic phlogopite | 10.000 | 10.000 | 10.000 | 10.000 | 10.000 |
| | Mica | 5.000 | 5.000 | 5.000 | 5.000 | 5.000 |
| | (Ca/Al) borosilicate | 2.000 | 2.000 | 2.000 | 2.000 | 2.000 |
| | Magnesium stearate | 1.000 | 1.000 | 1.000 | 1.000 | 1.000 |
| | Lauroyl lysine | 2.000 | 2.000 | 2.000 | 2.000 | 2.000 |
| | Starch | 5.000 | 5.000 | 5.000 | 5.000 | 5.000 |
| | Silica | 2.000 | 2.000 | 2.000 | 2.000 | 2.000 |
| | Red iron oxide | 0.030 | 0.030 | 0.030 | 0.030 | 0.030 |
| | Yellow iron oxide | 0.050 | 0.050 | 0.050 | 0.050 | 0.050 |
| | Chlorphenesin | 0.200 | 0.200 | 0.200 | 0.200 | 0.200 |
| Oil | Dimethicone | 3.000 | 3.000 | 3.000 | 3.000 | 3.000 |
| | Triethylhexanoin | 7.000 | 7.000 | 7.000 | 7.000 | 7.000 |
| | Sorbitan sesquiisostearate | 2.000 | 2.000 | 2.000 | 2.000 | 2.000 |
| | Tocopherol | q.s. | q.s. | q.s. | q.s. | q.s. |
| | Fragrance | q.s. | q.s. | q.s. | q.s. | q.s. |
| Total | | 100.000 | 100.000 | 100.000 | 100.000 | 100.000 |
| Evaluation | Roughness | S | A | B | C | C |
| | Smoothness | S | A | B | C | C |

From the above results, it is found that a sufficient improving effect can be obtained by adding niacinamide in an amount of about 5% by mass with respect to the total mass of a cosmetic. In addition to niacinamide, the cosmetic contains many solid particles of an extender pigment, a spherical powder, a coloring material, and the like, but it is found that when the average particle diameter of niacinamide to be blended is 40 µm or less, roughness and smoothness at the time of use are improved.

### [Examples 11 to 15]

A powder cosmetic (loose powder cosmetic) was prepared with a formulation presented in Table 3, and performance thereof was evaluated in a similar manner to Example 1.

### [Table 3]

**Table 3**

| Component | | Example 11 | Example 12 | Example 13 | Example 14 | Example 15 |
|---|---|---|---|---|---|---|
| Niacinamide | Pulverized product A (average particle diameter: 11 µm) | 5.000 | 2.000 | - | - | - |
| | Commercially available product X (average particle diameter: 55 µm) | - | - | 5.000 | 0.100 | - |
| Powder | Dimethicone hydrophobic treatment talc | Balance | Balance | Balance | Balance | Balance |
| | Mica | 45.000 | 45.000 | 45.000 | 45.000 | 45.000 |
| | Ba sulfate | 5.000 | 5.000 | 5.000 | 5.000 | 5.000 |
| | (Vinyldimethicone/ Methiconesilsesquioxane) crosspolymer | 5.000 | 5.000 | 5.000 | 5.000 | 5.000 |
| | Polymethyl methacrylate | 5.000 | 5.000 | 5.000 | 5.000 | 5.000 |
| | Chlorphenesin | 0.100 | 0.100 | 0.100 | 0.100 | 0.100 |
| Total | | 100.000 | 100.000 | 100.000 | 100.000 | 100.000 |
| Evaluation | Roughness | A | A | C | A | A |
| | Smoothness | A | A | B | A | A |

From the above results, it is found that even in the case of the loose powder cosmetic, by using pulverized niacinamide, a feeling of use excellent in roughness and smoothness can be achieved while the effect by niacinamide is obtained.

### [Examples 16 to 20]

A powder foundation cosmetic was prepared with a formulation presented in Table 4, and performance thereof was evaluated in a similar manner to Example 1.

### [Table 4]

**Table 4**

| Component | | Example 16 | Example 17 | Example 18 | Example 19 | Example 20 |
|---|---|---|---|---|---|---|
| Niacinamide | Pulverized product A (average particle diameter: 11 µm) | 5.000 | 2.000 | - | - | - |
| | Commercially available product X (average particle diameter: 55 µm) | - | - | 5.000 | 0.100 | - |
| Powder | Hydrophobic treatment talc | Balance | Balance | Balance | Balance | Balance |
| | Mica | 20.000 | 20.000 | 20.000 | 20.000 | 20.000 |
| | Starch | 10.000 | 10.000 | 10.000 | 10.000 | 10.000 |
| | Lauroyl lysine | 1.000 | 1.000 | 1.000 | 1.000 | 1.000 |
| | Silica | 6.000 | 6.000 | 6.000 | 6.000 | 6.000 |
| | Boron nitride | 5.000 | 5.000 | 5.000 | 5.000 | 5.000 |
| | Zinc oxide | 3.000 | 3.000 | 3.000 | 3.000 | 3.000 |
| | Dimethicone hydrophobic treatment titanium oxide | 15.000 | 15.000 | 15.000 | 15.000 | 15.000 |
| | Chlorphenesin | 0.200 | 0.200 | 0.200 | 0.200 | 0.200 |
| | Red iron oxide | 1.000 | 1.000 | 1.000 | 1.000 | 1.000 |
| | Yellow iron oxide | 2.000 | 2.000 | 2.000 | 2.000 | 2.000 |
| | Black iron oxide | 0.200 | 0.200 | 0.200 | 0.200 | 0.200 |
| Oil | Dimethicone | 3.000 | 3.000 | 3.000 | 3.000 | 3.000 |
| | Diisostearyl malate | 1.500 | 1.500 | 1.500 | 1.500 | 1.500 |
| | Triethylhexanoin | 2.000 | 2.000 | 2.000 | 2.000 | 2.000 |
| | Ethylhexyl methoxycinnamate | 3.000 | 3.000 | 3.000 | 3.000 | 3.000 |
| Total | | 100.000 | 100.000 | 100.000 | 100.000 | 100.000 |
| Evaluation | Roughness | A | A | C | A | A |
| | Smoothness | A | A | B | A | A |

From the above results, it is found that even in the case of the powder foundation cosmetic, by using pulverized niacinamide, a feeling of use excellent in roughness and smoothness can be achieved while the effect by niacinamide is obtained.

According to the present invention, it is possible to further provide cosmetics with the following formulations.

### [Formulation Example 1]

**[Table 5]**

| Table 5: Formulation Example of powder solid cosmetic | | |
|---|---|---|
| | Component | Blending ratio (% by mass) |
| Niacinamide | Niacinamide (pulverized product) | 5 |
| Inorganic powder | Metal soap hydrophobic treatment talc | balance |
| | Hydrophobic treatment synthetic phlogopite | 20 |
| | Synthetic phlogopite | 10 |
| | Mica | 5 |
| | Nε-Lauroyl-L-lysine | 2 |
| | Red iron oxide | 0.03 |
| | Yellow iron oxide | 0.05 |
| Organic powder | Polymethyl methacrylate | 5 |
| | (Vinyldimethicone/Methiconesilsesquioxane) crosspolymer | 2 |
| Antiseptic | Chlorphenesin | 0.2 |
| Oil | Dimethicone | 2 |
| | Triethylhexanoin | 5 |
| | Sorbitan sesquiisostearate | 1.5 |
| | Tocopherol | q.s. |
| | Fragrance | q.s. |
| Total | | 100 |

### [Formulation Example 2]

**[Table 6]**

| Table 6: Formulation Example of powder solid cosmetic | | |
|---|---|---|
| | Component | Blending ratio (% by mass) |
| Niacinamide | Niacinamide (pulverized product) | 5 |
| Inorganic powder | Dimethicone hydrophobic treatment talc | balance |
| | Mica | 45 |
| | Ba sulfate | 5 |
| Organic powder | (Vinyldimethicone/Methiconesilsesquioxane) crosspolymer | 5 |
| | Methyl methacrylate crosspolymer | 5 |
| Antiseptic | Chlorphenesin | 0.1 |
| Total | | 100 |

## Claims

1. A cosmetic comprising a powder of nicotinic acid or a derivative thereof having an average particle diameter of 40 µm or less.

2. The cosmetic according to claim 1, wherein a content of the powder is 3% by mass or more based on a total mass of the cosmetic.

3. The cosmetic according to claim 1 or 2, wherein the nicotinic acid or a derivative thereof is selected from nicotinic acid, niacinamide, benzyl nicotinate, tocopherol nicotinate, and a nicotinic acid β-butoxy ester.

4. The cosmetic according to claim 1 or 2, further comprising an inorganic powder different from the powder.

5. The cosmetic according to claim 1 or 2, wherein a content of water is 1% by mass or less based on a total mass of the cosmetic.

6. The cosmetic according to claim 1 or 2, wherein the cosmetic is in a form of a powder solid cosmetic or a powder cosmetic.

7. A cosmetic comprising a powder of nicotinic acid or a derivative thereof, wherein a content of the powder is 3% by mass or more based on a total mass of the cosmetic.

8. A method for manufacturing a cosmetic containing a powder of nicotinic acid or a derivative thereof having an average particle diameter of 40 µm or less, the method comprising
pulverizing particles of nicotinic acid or a derivative thereof having an average particle diameter of more than 40 µm to form a powder having an average particle diameter of 40 µm or less.

9. The method for manufacturing a cosmetic according to claim 8, wherein inorganic particles different from the particles are mixed with the particles, and then the obtained mixture is pulverized.
